(19) Europäisches Patentamt / European Patent Office / Office européen des brevets

(11) **EP 4 423 498 B1**

(12) **EUROPÄISCHE PATENTSCHRIFT**

(45) Veröffentlichungstag und Bekanntmachung des Hinweises auf die Patenterteilung:
**03.12.2025  Patentblatt 2025/49**

(21) Anmeldenummer: **22808805.0**

(22) Anmeldetag: **24.10.2022**

(51) Internationale Patentklassifikation (IPC):
**G01N 33/24** $^{(2006.01)}$    **B01D 53/62** $^{(2006.01)}$

(52) Gemeinsame Patentklassifikation (CPC):
**G01N 33/24; B01D 53/62**

(86) Internationale Anmeldenummer:
**PCT/EP2022/079669**

(87) Internationale Veröffentlichungsnummer:
**WO 2023/072879 (04.05.2023 Gazette 2023/18)**

(54) **VERFAHREN ZUR ERMITTLUNG EINER MENGE VON CHEMISCH GEBUNDENEM KOHLENSTOFFDIOXID UND VORRICHTUNG ZUR ERMITTLUNG DIESER MENGE**

METHOD FOR DETERMINING AN AMOUNT OF CHEMICALLY BOUND CARBON DIOXIDE AND DEVICE FOR DETERMINING SAID AMOUNT

PROCÉDÉ DE DÉTERMINATION D'UNE QUANTITÉ DE DIOXYDE DE CARBONE LIÉ CHIMIQUEMENT ET DISPOSITIF DE DÉTERMINATION DE LADITE QUANTITÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **25.10.2021  DE 102021127692**

(43) Veröffentlichungstag der Anmeldung:
**04.09.2024  Patentblatt 2024/36**

(73) Patentinhaber: Hemmersbach GmbH & Co. KG
**90489 Nürnberg (DE)**

(72) Erfinder: **PAESSLER, Dirk**
**90766 Fürth (DE)**

(74) Vertreter: **Lindner Blaumeier**
**Patent- und Rechtsanwälte**
**Partnerschaftsgesellschaft mbB**
**Dr. Kurt-Schumacher-Str. 23**
**90402 Nürnberg (DE)**

(56) Entgegenhaltungen:
- **BARGRIZAN SIMA ET AL: "Constraining the carbonate system in soils via testing the internal consistency of pH, pCO2 and alkalinity measurements", GEOCHEMICAL TRANSACTIONS, vol. 21, no. 4, 22 April 2020 (2020-04-22), XP093015397, DOI: 10.1186/ s12932-020-00069-5**
- **RYAN EDMUND M ET AL: "Modeling soil CO2 production and transport with dynamic source and diffusion terms: testing the steady-state assumption using DETECT v1.0", GEOSCIENTIFIC MODEL DEVELOPMENT, 28 May 2018 (2018-05-28), Katlenburg-Lindau, pages 1909 - 1928, XP093015402, Retrieved from the Internet <URL:https://gmd.copernicus.org/ articles/11/1909/2018/gmd-11-1909-2018.pdf> [retrieved on 20230118], DOI: 10.5194/ gmd-11-1909-2018**
- **HEIMSCH LAURA ET AL: "Carbon dioxide fluxes and carbon balance of an agricultural grassland in southern Finland", BIOGEOSCIENCES, vol. 18, no. 11, 10 June 2021 (2021-06-10), pages 3467 - 3483, XP093015400, DOI: 10.5194/ bg-18-3467-2021**

- **YANG WENZHU ET AL: "Absorbed carbon dioxide in saline soil from northwest China", CATENA, ELSEVIER, AMSTERDAM, NL, vol. 207, 24 August 2021 (2021-08-24), XP086795131, ISSN: 0341-8162, [retrieved on 20210824], DOI: 10.1016/J.CATENA.2021.105677**

**Beschreibung**

Die Erfindung betrifft ein Verfahren zur Ermittlung einer Menge von innerhalb eines Zeitintervalls mithilfe eines erdbodenseitigen Reaktionsmittels chemisch gebundenem Kohlenstoffdioxid. Daneben betrifft die Erfindung eine Vorrichtung zur Ermittlung dieser Menge.

Kohlenstoffdioxidausstoß führt zu höheren Kohlenstoffdioxidkonzentrationen in der Atmosphäre und somit aufgrund des Treibhauseffektes zu einem langfristigen Temperaturanstieg. In einem mittleren Szenario, in dem davon ausgegangen wird, dass der Ausstoß von Kohlenstoffdioxid ab ungefähr der Mitte des Jahrhunderts aufgrund von Klimaschutzmaßnahmen wieder sinkt, wird in aktuellen Modellrechnungen von einem Temperaturanstieg von ca. 2,7°C bis zum Ende des Jahrhunderts ausgegangen. Gelingt eine Reduktion des Kohlenstoffdioxidausstoßes hingegen nicht oder erst zu einem deutlich späteren Zeitpunkt, sind auch erheblich höhere Temperaturerhöhungen möglich. Entsprechende Temperaturerhöhungen führen neben anderen Problemen auch zu einem deutlichen Anstieg des Meeresspiegels, der langfristig deutlich oberhalb eines Meters liegen kann.

Wesentlich für die Beschränkung des Temperaturanstiegs und die Vermeidung bzw. Reduzierung von dessen negativen Auswirkungen ist es, die Nutzung von fossilen Brennstoffen und den damit verbundenen Kohlenstoffdioxidausstoß zu reduzieren. Da es jedoch sehr herausfordernd ist, alleine durch Reduzierung des Kohlenstoffdioxidausstoßes ein kohlenstoffdioxidneutrales Wirtschaften zu erreichen, und da eine Reduzierung des bereits in der Atmosphäre vorhandenen Kohlenstoffdioxids allein durch natürliche Prozesse sehr langwierig ist, ist es zweckmäßig, Maßnahmen zu nutzen, die Kohlenstoffdioxid aus der Luft entnehmen und binden.

Ein vielversprechender Ansatz hierfür ist die Verbesserung bzw. Verstärkung von natürlichen Verwitterungsprozessen, in denen bestimmte Gesteinsorten in Anwesenheit von Kohlenstoffdioxid und Wasser zersetzt werden, wobei Teile des an der Reaktion beteiligten Kohlenstoffdioxids durch Bildung von Karbonaten chemisch gebunden werden. Da solche Verwitterungsprozesse an der Gesteinsoberfläche ablaufen, sind sie üblicherweise sehr langsam. Es ist jedoch bekannt, dass durch eine deutliche Oberflächenvergrößerung, beispielsweise in dem das Gestein zunächst zu feinem Staub zermahlen wird, eine deutlich beschleunigte Verwitterung und somit Kohlenstoffdioxidbindung erreicht werden kann. Dies wir auch als "enhanced weathering" bezeichnet.

Beispielsweise können Mineralien aus der Olivingruppe pro Tonne zwischen 0,5t und 1,5t Kohlenstoffdioxid binden. Auch andere Gesteinssorten, beispielsweise Basalt, Dunit, Kimberlit oder Wollastonit, sind zu diesem Zweck geeignet. Die Nutzung einer beschleunigten Verwitterung zur Kohlenstoffdioxidbindung wird beispielsweise in der Druckschrift WO 2020/263910 A1 diskutiert.

Eine Modellierung der Wechselwirkung zwischen Kohlenstoffdioxid und Böden ist Gegenstand von BARGRIZAN SIMA ET AL: "Constraining the carbonate system in soils via testing the internal consistency of pH, pCO2 and alkalinity measurements", GEOCHEMICAL TRANSACTIONS, Bd. 21, Nr. 4, 22. April 2020, XP093015397, DOI: 10.1186/ s12932-020-00069-5.

Da es sich bei den genannten Mineralien bzw. Gesteinssorten zum Teil um zulässige Düngemittel handelt, würden prinzipiell sehr große Flächen für eine solche beschleunigte Verwitterung bereitstehen, sodass viel Kohlenstoffdioxid gebunden werden könnte und somit ein erheblicher Beitrag zur Minderung des Klimawandels geleistet werden könnte.

Um diese zu erreichen, muss diese Flächennutzung jedoch für Landwirte und Grundbesitzer attraktiv sein. Dies könnte dadurch erreicht werden, dass aufgrund der negativen Emission durch die Kohlenstoffdioxidbindung durch Landwirte bzw. Flächenbesitzer Emissionszertifikate verkauft werden können, um Kohlenstoffdioxidemissionen zu kompensieren. Hierzu wäre es jedoch erforderlich, die gebundene Kohlenstoffdioxidmenge zu quantifizieren. Da bei einer beschleunigten Verwitterung auf freiem Feld jedoch deutlich andere Verwitterungsgeschwindigkeiten resultieren können als im Labor, sind hierfür Ansätze erforderlich, die tatsächlich gebundene Kohlenstoffdioxidmenge zu quantifizieren.

Wird, wie in der obig genannten Druckschrift, davon ausgegangen, dass das gebundene Kohlenstoffdioxid, beispielsweise durch Temperatureinwirkung, wieder vom verwitterten Gestein getrennt wird, wäre es möglich, die gebundene Kohlenstoffdioxidmenge unmittelbar zu messen. Diese wäre jedoch sehr aufwendig, da hierzu das Gestein wieder von großen Feldern bzw. Freiflächen eingesammelt und aufwendig nachverarbeitet werden müsste. Zudem müsste das vom verwitterten Gestein abgeschiedene Kohlenstoffdioxid anschließend auf andere Weise gespeichert werden. Wird hingegen davon ausgegangen, dass das verwitterte Gestein unmittelbar zur langfristigen Bindung von Kohlenstoffdioxid genutzt wird, ist eine solche direkte Messung nicht möglich.

Der Erfindung liegt somit die Aufgabe zugrunde, die Menge eines durch ein erdbodenseitiges Reaktionsmittel, beispielsweise durch die obig genannten Gesteins- bzw. Mineralsorten, chemisch gebundenen Kohlenstoffdioxids zu ermitteln, wobei dies insbesondere ohne ein Aufbrechen dieser chemischen Bindung erfolgen soll.

Die Aufgabe wird erfindungsgemäß durch ein Verfahren zur Ermittlung einer Menge von innerhalb eines Zeitintervalls mithilfe eines erdbodenseitigen Reaktionsmittels chemisch gebundenem Kohlenstoffdioxid gelöst, dass die folgenden Schritte umfasst:

- Einlesen wenigstens eines das Reaktionsmittel betreffenden Reaktionsmittelparameters,
- Erfassen eines jeweiligen Messwertes für die Leitfähigkeit des Erdbodens und/oder für eine jeweilige Kationenkonzentration wenigstens eines Kations im Erdboden durch wenigstens einen in oder an dem Erdboden angebrachten Sensor, und
- Ermittlung der Menge des gebundenen Kohlenstoffdioxids in Abhängigkeit sowohl des Reaktionsmittelparameters als auch des Messwertes oder der Messwerte.

Im Rahmen der Erfindung wurde erkannt, dass die Menge des gebundenen Kohlenstoffdioxids mit guter Genauigkeit ermittelt werden kann, wenn einerseits durch den Reaktionsmittelparameter die Eigenschaften des Reaktionsmittels, also beispielsweise dessen Korngröße und/oder Gehalt bestimmter Mineralien, berücksichtigt wird, und andererseits wenigstens ein Messwert berücksichtigt wird, der die konkreten Bedingungen der Verwitterung vor Ort betrifft.

Der Reaktionsmittelparameter kann beispielsweise die Menge des gebundenen Kohlenstoffdioxids für eine bestimmte Menge Reaktionsmittel bzw. die Zeitableitung hiervon, also eine Bindungsrate, beschreiben. Ein solcher Reaktionsmittelparameter kann beispielsweise ermittelt werden, indem für Stichproben des Reaktionsmittels eine Verwitterung unter Laborbedingungen erfolgt, wodurch die genaue Menge des aufgenommenen Kohlenstoffdioxids bzw. die Aufnahmerate unter diesen Bedingungen bestimmt werden kann. Es ist jedoch auch möglich, dass als Reaktionsmittelparameter wenigstens eine andere Information, beispielsweise eine Korngröße und/oder ein Mineraliengehalt des Reaktionsmittels, eingelesen wird, wobei beispielsweise Kennfelder oder bekannte, beispielsweise durch Regression bestimmte, mathematische Zusammenhänge genutzt werden können, um hieraus die Menge des bindbaren Kohlenstoffdioxids pro Menge Reaktionsmittel, eine Bindungsrate oder Ähnliches zu ermitteln.

Wie später noch im Detail erläutert werden wird, hängt die durch ein Reaktionsmittel innerhalb eines Zeitintervalls gebundene Menge von Kohlenstoffdioxid bzw. die Bindungsrate von einer Vielzahl von Faktoren ab. Im Rahmen der Erfindung wurde jedoch erkannt, dass bereits durch Berücksichtigung der Leitfähigkeit des Erdbodens bzw. der Kationenkonzentration wenigstens eines Kations eine erhebliche Verbesserung der Genauigkeit der ermittelten Menge des gebundenen Kohlenstoffdioxids gegenüber der ausschließlichen Berücksichtigung des Reaktionsmittelparameters erreicht werden kann. Je nach Anwendungsfall kann es somit bereits ausreichend sein, einen oder mehrere der genannten Messwerte bei der Ermittlung des gebundenen Kohlenstoffdioxids zu berücksichtigen. Andererseits kann es in anderen Anwendungsfällen zweckmäßig sein, die Genauigkeit der Ermittlung weiter zu verbessern, indem, wie später noch erläutert wird, zusätzliche Informationen berücksichtigt werden.

Ein wesentlicher Vorteil des erfindungsgemäßen Verfahrens ist es, dass durch eine sensorische Erfassung von Messwerten unmittelbar am Reaktionsort bzw. Verwitterungsort eine Zeitauflösung der Messwerterfassung und somit der Ermittlung der Kohlenstoffdioxidbindung erreicht werden kann, die durch andere Ansätze zur Berücksichtigung von lokalen Einflüssen auf die Verwitterung, beispielsweise durch eine Entnahme und Untersuchung von Bodenproben, nicht mit vertretbarem Aufwand erreicht werden kann. Beispielsweise ist durch Nutzung von drahtlos ausgelesenen Sensoren problemlos eine Aktualisierung der Messwerte mehrmals pro Tag oder sogar mehrmals pro Stunde möglich, sodass die lokale Reaktionsumgebung erheblich besser abgebildet werden kann, als wenn beispielsweise nur wöchentlich oder monatlich Bodenproben entnommen würden.

Für den Messwert bzw. die Messwerte können insbesondere zeitliche Verläufe erfasst werden, wodurch insbesondere ein zeitlicher Verlauf der Menge des pro Zeitintervall gebundenen Kohlenstoffdioxids bestimmt werden kann. Durch ein Zeitintegral bzw. Summenbildung über die Messintervalle in denen Messungen erfolgen, kann dann die in dem gesamten Zeitintervall gebundene Menge von Kohlenstoffdioxid bestimmt werden.

Geeignete Sensoren zur Ermittlung der obig genannten Messwerte bzw. der später noch diskutierten, zusätzlich berücksichtigbaren Sensordaten, sind an sich aus dem Bereich der intelligenten Landwirtschaft bekannt. Beispielsweise beschreibt die Internetseite "https://www.dragino.com/products/lora-lorawan-endnode/item/159-Ise01.html" einen Sensor zur Überwachung der Bodenfeuchtigkeit, Temperatur und Leitfähigkeit. Die Internetseite "https://teralytic.com/how-it-works.html" offenbart beispielsweise Messungen von Salzgehalt, pH-Wert und Konzentrationen von bestimmten Ionen. Die Anpassung der Selektivität für bestimmte Ionen bzw. Kationen ist beispielsweise durch Wahl einer geeigneten ionenselektiven Elektrode möglich.

Die Menge des gebundenen Kohlenstoffdioxids kann in Abhängigkeit von Sensordaten des Sensors und/oder wenigstens eines weiteren Sensors ermittelt werden, wobei die Sensordaten einen vertikalen Wasserfluss im Erdboden und/oder eine Niederschlagsmenge und/oder eine Feuchtigkeit im Erdboden und/oder eine Alkalinität und/oder einen Näherstoffgehalt des Erdbodens und/oder einen Partialdruck von Kohlenstoffdioxid in der Luft oberhalb des Erdbodens betreffen. Durch Nutzung aller oder auch von nur Teilen der genannten Sensordaten kann die Genauigkeit der Ermittlung der Menge des gebundenen Kohlenstoffdioxids weiter verbessert werden.

Die Sensordaten können hierbei lokal durch einen im Bereich des Erdbodens bzw. innerhalb des Erdbodens angebrachten Sensor erfasst werden. Beispielsweise für die Niederschlagsmenge kann es jedoch vorteilhaft sein, stattdessen den Sensor eines Satelliten zu nutzen, wobei in diesem Fall die Zuordnung der Sensordaten zu dem Gebiet, für das die Menge des gebundenen Kohlenstoffdioxids ermittelt werden soll, beispielsweise dadurch erfolgen kann, dass der Ort des Gebiets gespeichert ist bzw., wie später noch erläutert wird, beispielsweise aufgrund von GPS-Daten bestimmt wird. Der Partialdruck von Kohlenstoffdioxid kann insbesondere bodennah ermittelt werden, beispielsweise indem ein Messmodul, das beispielsweise teilweise in den Erdboden eingesteckt ist, in seinem über den Erdboden hinausragenden Abschnitt einen geeigneten Sensor aufweist.

Der vertikale Wasserfluss in Erdboden und/oder eine Niederschlagsmenge können insbesondere durch ein Lysimeter gemessen werden. Beispielsweise kann ein Lysimeter auf Basis von vertikal beabstandeten Feuchtigkeitssensoren verwendet werden, wodurch ein wartungsarmer Betrieb und eine elektronische Auslesung besonders einfach umsetzbar sind.

Eine direkte Messung der Alkalinität kann relativ aufwendig sein. Hierzu könnte beispielsweise automatisiert eine Probe des Erdbodens entnommen und untersucht werden. Es kann daher vorteilhaft sein, die Alkalinität stattdessen auf Basis von weiteren Sensordaten bzw. Messwerten zu ermitteln bzw. abzuschätzen, beispielsweise indem eine aufgrund von anderen Parametern erwartete Änderung des pH-Werts mit einer tatsächlichen pH-Wertänderung verglichen wird. Hierzu kann es zweckmäßig sein, von externen Quellen zusätzliche Daten bereitzustellen, die beispielsweise eine Art der Bepflanzung, bestimmte Bodeneigenschaft oder Ähnliches beschreiben. Diese Zusatzdaten können entweder zum Ort des Sensors bzw. der Sensoren übertragen werden oder die Berechnung kann beispielsweise auf einem Server oder einer anderen vom Sensor beabstandeten Datenverarbeitungseinrichtung erfolgen.

Sensordaten, die den Näherstoffgehalt des Erdbodens betreffen können insbesondere die Konzentration von Stickstoff-, Kalium- bzw. Phosphorverbindungen bzw. -ionen im Erdboden betreffen.

Die Menge des gebundenen Kohlenstoffdioxids kann in Abhängigkeit von Sensordaten des Sensors und/oder des weiteren Sensors und/oder wenigstens eines weiteren Sensors ermittelt werden, wobei die Sensordaten einen pH-Wert und/oder eine Temperatur des Erdbodens betreffen. Auch der pH-Wert und die Temperatur des Erdbodens können die Bindung von Kohlenstoffdioxid beeinflussen bzw. die Entstehung von Kohlensäure im Rahmen des Bindungsprozesses kann auf Basis des pH-Wertes erkannt werden.

Insbesondere können mehrere oder sogar alle der verschiedenen obig genannten Sensordaten bei der Ermittlung der Menge des gebundenen Kohlenstoffdioxids berücksichtigt werden. Durch Auswertung eines breiten Spektrums von gemessenen Parametern, die die Verwitterung beeinflussen und/oder durch diese beeinflusst werden, kann eine hohe Genauigkeit für die Ermittlung der Menge des gebundenen Kohlenstoffdioxids erreicht werden, wobei aufgrund der sensorischen Erfassung eine hohe Zeitauflösung und bei Nutzung von mehreren beabstandeten Sensoren zudem eine hohe Ortsauflösung erreicht werden kann.

Der Reaktionsmittelparameter kann einen Grundwert für die Menge des gebundenen Kohlenstoffdioxids oder für die Zeitableitung dieser Menge vorgeben, der im Rahmen der Berechnung mit wenigstens einem Korrekturfaktor multipliziert wird, wobei wenigstens einer der Korrekturfaktoren jeweils von dem Messwert oder wenigstens einem der Messwerte und/oder von zumindest Teilen der Sensordaten abhängt. Es hat sich herausgestellt, dass bei einer solchen Berechnung Korrekturen aufgrund von unterschiedlichen Einflüssen besonders gut separierbar sind. Zumindest einer der Korrekturfaktoren kann sowohl von zumindest einer der genannten Größen als auch von wenigstens einer der später noch erläuterten Vorabinformationen abhängen. Einzelne Korrekturfaktoren können auch ausschließlich von wenigstens einer der Vorabinformationen abhängen.

Als Beispiel kann eine Verwitterungsrate $R_{real}$ zum Zeitpunkt t, die der Zeitableitung der Menge des gebundenen Kohlenstoffdioxids entsprechen oder zu dieser proportional sein kann, als Produkt aus dem Grundwert $R_{Grund}$ für diese Zeitableitung und 6 Korrekturfaktoren berechnet werden:

$$R_{real}(t) = R_{Grund}(t) * f_{Leitfähigkeit}(t) * f_{Boden}(t) * f_{Umwelt}(t) * f_{Flüssigkeit}(t) * f_{pH}(t) * f_{alk}(t)$$

Der Grundwert $R_{Grund}$ kann wie obig erläutert beispielsweise unter Laborbedingungen bestimmt sein. Die verschiedenen Korrekturfaktoren f können jeweils von wenigstens einem der Messwerte und/oder von Sensordaten und/oder von später noch erläuterten Vorabinformationen abhängen. Zur Ermittlung des jeweiligen Korrekturfaktors f aus den jeweiligen Eingangsgrößen können beispielsweise Versuchsserien durchgeführt werden, um einen solchen Zusammenhang beispielsweise als Look-up-Tabelle oder durch eine Regressionsanalyse oder auch durch Maschinenlernen zu ermitteln.

Der Korrekturfaktor $f_{Leitfähigkeit}$ kann z.B. ausschließlich von dem Messwert für die Leitfähigkeit des Erdbodens abhängen. Der Korrekturfaktor $f_{Boden}$ kann weitere Eigenschaften des Bodens betreffen und insbesondere von dem wenigstens einen Messwert für die Kationenkonzentration und/oder von Vorabinformationen, die insbesondere eine Porosität und/oder einen Bodentyp des Erdbodens und/oder im Erdboden vorhandene Pilze und/oder Bakterien und/oder eine Bepflanzung des Erdbodens betreffen, abhängen. Der Korrekturfaktor $f_{Umwelt}$ kann Umwelteinflüsse betreffen und insbesondere von einer als Teil der Sensordaten gemessenen Temperatur und/oder Niederschlagsmenge bzw. von diese Größen betreffenden Vorabinformation abhängen. Der Korrekturfaktor $f_{pH}$ wird aus einem insbesondere als Sensordaten erfassten pH-Wert des Bodens ermittelt und der Korrekturfaktor $f_{alk}$ aus einer Alkalinität, die als Sensordaten erfasst oder als Vorabinformation bereitgestellt werden kann. Die Nutzung der sechs oben genannten Korrekturfaktoren ist nur ein mögliches Beispiel für eine vorteilhafte Korrekturfaktorkombination. Es ist z.B. auch möglich, einen oder mehrere der genannten Korrekturfaktoren nicht zu berücksichtigen oder weitere Korrekturfaktoren heranzuziehen.

Die einzelnen Korrekturfaktoren f schwanken typischerweise um den Wert 1 und bringen jeweils eine gewisse Erhöhung bzw. Erniedrigung der Zeitableitung der Menge des gebundenen Kohlenstoffdioxids durch die Einflussfaktoren, von denen sie abhängen, zum Ausdruck. Es ist zwar nicht notwendig, alle genannten Korrekturfaktoren f zu nutzen, die Berücksichtigung einer Vielzahl von Einflussfaktoren kann jedoch insgesamt die Genauigkeit der ermittelten Verwitterungsrate $R_{real}$ verbessern.

Die Menge der innerhalb eines Zeitintervalls mithilfe eines erdbodenseitigen Reaktionsmittels chemisch gebundenen Kohlenstoffdioxids kann analytisch durch Integration der Verwitterungsrate $R_{real}$ über die Zeit ermittelt werden. Da die verschiedenen Einflussgrößen, also insbesondere der Messwert bzw. die Messwerte und die Sensordaten, jedoch typischerweise ohnehin zeitdiskret erfasst werden, kann ein solches Integral näherungsweise durch Summation über die Messintervalle berechnet werden. Hierbei ist es nicht notwendig, dass für alle Einflussfaktoren das gleiche Messintervall genutzt wird.

Als Kationenkonzentration kann die Konzentration von Natriumionen und/oder von Kalziumionen und/oder von Magnesiumionen erfasst werden. Insbesondere kann als Kationenkonzentration die Konzentration jenes Kations bzw. jener Kationen ermittelt werden, die vor der Verwitterung bzw. Reaktion Teile des Reaktionsmittels waren.

Die Verwitterungsreaktion wird im Folgenden am Beispiel der Verwitterung von Forsterit ($Mg_2SiO_4$) erläutert. Hierfür kann folgende Reaktionsgleichung angegeben werden:

$$Mg_2SiO_4 + 4CO_2 + 4H_2O \leftrightarrow 2Mg^{2+} + 4HCO_3^- \leftrightarrow 2MgCO_3 + SiO_2 + 2Co_2 + 4H_2O$$

Wie an dieser Reaktionsgleichung zu erkennen ist, werden durch die Verwitterung des Forsterits in Anwesenheit von Kohlenstoffdioxid und Wasser die Hälfte der eingesetzten Kohlenstoffdioxidmoleküle als Magnesiumkarbonat gebunden. In einem Zwischenschritt dieser Reaktion liegen gelöste Magnesiumkationen vor, so dass die Konzentration dieser Kationen mit der Verwitterungsrate korreliert.

Der jeweilige Messwert und/oder die Sensordaten und/oder wenigstens ein aus dem Messwert und/oder den Sensordaten ermitteltes Zwischenergebnis kann von einer Messposition, an der der jeweilige Sensor oder die jeweiligen Sensoren angeordnet ist oder sind, drahtlos an eine Verarbeitungseinrichtung übertragen werden, die die Ermittlung der Menge des gebundenen Kohlenstoffdioxids durchführt. Die Kommunikation kann beispielsweise durch ein Mobilfunkprotokoll, z. B. 3G, LTE, 5G, Edge, etc., oder durch ein Wide Area Network, insbesondere ein Long Range Wide Area Network (LoRaWAN, z. B. Zero-G) implementiert werden.

Die Nutzung einer von der jeweiligen Messposition beabstandeten Verarbeitungseinrichtung ist insbesondere dann zweckmäßig, wenn bei der Ermittlung der Menge des gebundenen Kohlenstoffdioxids Messwerte bzw. Sensordaten von mehreren verschiedenen Messpositionen, beispielsweise von verschiedenen landwirtschaftlich genutzten Feldern, die jeweils eine eigene Sensorik aufweisen, gesammelt und gemeinsam ausgewertet werden sollen. Dies kann beispielsweise zweckmäßig sein, um für die Messwerte bzw. die Sensordaten nicht nur eine hohe zeitliche Auflösung, sondern auch eine hohe räumliche Auflösung zu erreichen und somit die Genauigkeit der Ermittlung weiter zu verbessern.

Die Verarbeitungseinrichtung kann beispielsweise ein Server, eine Cloud-basierte Lösung oder auch ein Arbeitsplatzrechner sein. Die Nutzung einer separaten Verarbeitungseinrichtung kann auch zweckmäßig sein, wenn beispielsweise Vorabinformationen im Rahmen der Ermittlung der gebundenen Kohlenstoffdioxidmenge herangezogen werden sollen, die in dieser Verarbeitungseinrichtung gespeichert sind oder von externen Quellen, beispielsweise einem Labor, das Bodenproben untersucht, z.B. über das Internet, bezogen werden sollen.

Die Verarbeitungseinrichtung selbst oder eine von dieser mit Informationen bezüglich der Menge des gebundenen Kohlenstoffdioxids versorgte weitere Einrichtung kann dies Informationen beispielsweise über ein Webinterface, eine App oder Ähnliches an Nutzer bereitstellen. Hierbei können kurze Verzögerungen von deutlich weniger als einer Stunde, insbesondere von weniger als 10 Minuten, zwischen der Erfassung der Messwerte bzw. der Sensordaten und der Bereitstellung einer aktualisierten Menge von gebundenem Kohlenstoffdioxid erreicht werden, so dass eine just-in-time-Information von beispielsweise landbereitstellenden Landwirten oder ähnlichen Verfahrensteilnehmern erfolgen kann.

In dem erfindungsgemäßen Verfahren können mehrmals täglich oder mehrfach pro Stunde der Messwert oder die Messwerte und/oder zumindest Teile der Sensordaten erfasst werden und in Abhängigkeit hiervon die Menge des gebundenen Kohlenstoffdioxids ermittelt werden. Durch eine hohe Zeitauflösung der Messung und Ermittlung wird eine hohe Genauigkeit erreicht, die auch kurzfristige Änderungen der Kohlenstoffdioxidaufnahme, beispielsweise aufgrund von kurzen Niederschlägen, erkennen und berücksichtigen kann. Eine derart hohe Zeitauflösung der Messdatenerfassung und Auswertung wäre beispielsweise bei einer Ermittlung der Menge des gebundenen Kohlenstoffdioxids ausschließlich auf Basis von entnommenen Bodenproben nicht mit vertretbarem Aufwand realisierbar.

Der Messwert oder wenigstens einer der Messwerte und/oder die Sensordaten können an mehreren Messpositionen durch einen jeweiligen Sensor erfasst werden, wobei die Messpositionen insbesondere weniger als 500 m oder weniger als 200 m voneinander beabstandet sind. Beispielsweise kann zumindest für Teile der Messwerte bzw. Sensordaten ein separater Sensor bzw. ein separates Messmodul, das mehrere Sensoren umfasst, pro Feld bereitgestellt werden. Es kann somit beispielsweise eine separate Messdatenerfassung pro halben Hektar oder pro Hektar erfolgen. Dies ist nicht nur vorteilhaft, um eine individuelle Abrechnung auch bei relativ kleinen Feldern verschiedener Landwirte oder in ähnlichen Situationen zu ermöglichen, es ermöglicht auch insgesamt eine Verbesserung der Mengenerfassung, da z.B. lokale Unterschiede bezüglich der Bepflanzung, Bodenbeschaffenheit etc. mit guter Genauigkeit berücksichtigt werden können.

Dem Boden kann, insbesondere im Bereich einer jeweiligen Messposition, an der der jeweilige Sensor oder die jeweiligen Sensoren angeordnet ist oder sind, vor Erfassung des jeweiligen Messwertes und/oder der Sensordaten eine Bodenprobe entnommen werden, die, insbesondere beabstandet von dem Entnahmeort in einem Labor, untersucht wird, um eine Alkalinität und/oder eine Porosität und/oder einen Bodentyp des Erdbodens und/oder im Erdboden vorhandene Pilze und/oder Bakterien als Vorabinformation zu ermitteln, wobei die Menge des gebundenen Kohlenstoffdioxids in Abhängigkeit der Vorabinformation ermittelt wird. Als Bodentyp kann beispielsweise ermittelt werden, ob es sich um einen sandigen oder lehmigen Boden handelt oder Ähnliches.

Wie bereits erläutert, ist eine Entnahme von Bodenproben typischerweise nicht mit der gleichen Häufigkeit möglich, wie eine Messung von Messwerten bzw. Sensordaten durch Sensoren. Zugleich können mithilfe von Bodenproben und beispielsweise Laboruntersuchungen die genannten Parameter jedoch genauer bzw. überhaupt erst ermittelt und somit berücksichtigt werden. Es kann daher vorteilhaft sein, in relativ großen Zeitabständen von beispielsweise einigen Wochen oder sogar Monaten Bodenproben zu entnehmen, um Informationen über die genannten, relativ langsam veränderlichen Parameter des Bodens zu ermitteln, während schneller veränderliche Parameter sensorisch erfasst und berücksichtigt werden können.

Eine jeweilige Vorabinformation, die insbesondere eine Alkalinität und/oder eine Porosität und/oder einen Bodentyp des Erdbodens und/oder im Erdboden vorhandene Pilze und/oder Bakterien und/oder eine Bepflanzung des Erdbodens und/oder das Wetter betrifft, und/oder die jeweilige auf Basis der Bodenprobe bestimmte Vorabinformation kann für mehrere Gebiete separat bereitgestellt werden, wobei in einem Messgebiet, das den und/oder den weiteren Sensor umfasst, zusätzliche eine Positionsbestimmungseinrichtung verwendet wird, die eine das jeweilige Messgebiet betreffende Positionsinformation bereitstellt, wobei auf Basis der Positionsinformation die dem Messgebiet zugeordnete Vorabinformation ausgewählt wird, wonach in Abhängigkeit von dieser und dem wenigstens einen in dem Messgebiet erfassten Messwert und/oder von zumindest Teilen der in dem Messgebiet erfassten Sensordaten die Menge des gebundenen Kohlenstoffdioxids oder ein Zwischenergebnis, von dem die ermittelte Menge des gebundenen Kohlenstoffdioxids abhängt, bestimmt wird. Als Zwischenergebnis kann insbesondere eine Zeitableitung bzw. eine zeitliche Änderung der Menge bestimmt werden, aus der durch Integration bzw. zeitdiskrete Summation die Menge bestimmt werden kann.

Die Vorabinformation kann durch eine Bodenprobe bestimmt sein oder auch manuell eingegeben sein, beispielsweise wenn sie eine Bepflanzung betrifft. Ergänzend oder alternativ kann als Vorabinformation beispielsweise eine Information genutzt werden, die per Satellit, durch ein flugzeuggestützten Sensor oder Ähnliches ermittelt werden kann.

Alle Sensoren für einen bestimmten Messbereich oder zumindest Teile dieser Sensoren können gemeinsam mit der Positionsbestimmungseinrichtung in eine gemeinsame Messstation integriert sein. Es ist jedoch auch möglich, dass mehrere verteilt angeordnete Sensoren, beispielsweise über ein drahtloses lokales Netzwerk, mit einer Zentralstation für das jeweilige Messgebiet kommunizieren, die eine Positionsbestimmungseinrichtung aufweist oder mit dieser ebenfalls kommuniziert.

Wird die Menge bzw. das Zwischenergebnis für mehrere Messgebiete ermittelt, können die Mengen bzw. die Zwischenergebnisse aufaddiert werden bzw. es kann eine gewichtete Summation erfolgen, um beispielsweise unterschiedliche Größen der Messgebiete zu berücksichtigen.

Es kann zweckmäßig sein, verschiedene Zwischenergebnisse, beispielsweise unterschiedliche der obig erläuterten Korrekturfaktoren, für unterschiedlich große Gebiete zu ermitteln. Beispielsweise können Niederschlagsmengen bzw. Temperaturen für relativ große Messgebiete erfasst werden und eine Leitfähigkeit des Erdbodens bzw. eine Kationenkonzentration, eine Nährstoffkonzentration oder Ähnliches sowie die daraus resultierenden Korrekturfaktoren können als Zwischenergebnis für mehrere Teilgebiete dieses größeren Messgebiets separat ermittelt werden.

Die Ermittlung der Positionsinformation ermöglicht es auch, die Menge des gebundenen Kohlenstoffdioxids für mehrere separate Messgebiete, z.B. für verschiedene Felder, durch eine gemeinsame Verarbeitungseinrichtung zu realisieren. Optional kann hierbei dennoch eine separate Abrechnung bzw. Zertifizierung der Kohlenstoffdioxidbindung für die einzelnen Messgebiete oder Untergruppen der Messgebiete möglich sein.

Die Positionsbestimmungseinrichtung kann beispielsweise eine satellitenbasierte Positionsbestimmung, z.B. auf GPS-Basis, durchführen. Alternativ zur Nutzung einer Positionsbestimmungseinrichtung wäre es beispielsweise auch möglich, die Messwerte bzw. Sensordaten von Sensoren eines Messgebiets über eine jeweilige Kommunikationseinrichtung an die Verarbeitungseinrichtung bereitzustellen und hierbei eine Kennung der Kommunikationseinrichtung, beispielsweise eine Teilnehmernummer in einem Drahtlosnetzwerk, mit zu erfassen. Die Zuordnung der Messwerte bzw. Sensordaten zu bestimmten Messgebieten kann dann auf Basis dieser Kennung und einer vorangehend vorgegebenen Zuordnung der Kennungen zu den Messgebieten erfolgen. Hierzu wäre es jedoch erforderlich, manuell die einzelnen Kommunikationseinrichtungen den einzelnen Messgebieten zuzuweisen, so dass ein höherer Aufwand resultiert und ein gewisses Fehlerrisiko, beispielsweise durch ein versehentliches Vertauschen von Kommunikationseinrichtungen bzw. Messmodulen, verbleibt.

Neben dem erfindungsgemäßen Verfahren betrifft die Erfindung eine Vorrichtung zur Ermittlung einer Menge von innerhalb eines Zeitintervalls mithilfe eines erdbodenseitigen Reaktionsmittels chemisch gebundenem Kohlenstoffdioxid, umfassend wenigstens einen Sensor zur Erfassung von Messwerten für die Leitfähigkeit des Erdbodens und/oder für eine jeweilige Kationenkonzentration wenigstens eines Kations im Erdboden und eine Verarbeitungseinrichtung, wobei die Vorrichtung zur Durchführung des erfindungsgemäßen Verfahrens eingerichtet ist.

Als Verarbeitungseinrichtung kann insbesondere eine übliche Datenverarbeitungseinrichtung, beispielsweise ein Server, ein Arbeitsplatzrechner, eine Cloud-basierte Datenverarbeitungseinrichtung, ein Smartphone, ein Tablett oder Ähnliches genutzt werden, die die Messdaten und insbesondere die obig erläuterten Sensordaten von dem Sensor oder den Sensoren empfängt und den Reaktionsmittelparameter und optional die Vorabinformation vorhält bzw., z. B. über das Internet, von einer weiteren Einrichtung abruft. Die Ermittlung der Menge des gebundenen Kohlenstoffdioxids und die Datenerfassung vom Sensor, der optional genutzten Positionsbestimmungseinrichtungen, etc., kann beispielsweise durch ein Programm implementiert sein, das durch die Datenverarbeitungseinrichtung ausgeführt wird.

Die Vorrichtung kann wenigstens ein Messmodul umfassen, das ein Gehäuse, das zur Einführung in den Erdboden ausgebildet ist, wenigstens einen Sensor, eine Kommunikationseinrichtung zur drahtlosen Übertragung von mittels des Sensors erfassten Messwerten und/oder Sensordaten und/oder eines aus dem Messwert und/oder den Sensordaten ermittelten Zwischenergebnisses an die Verarbeitungseinrichtung und wenigstens eine Energieversorgungseinrichtung zur Versorgung der Kommunikationseinrichtung und des Sensors umfassen. Das Messmodul kann zumindest in einem Abschnitt stab- bzw. pfahlförmig sein, um ein leichtes Einbringen in den Erdboden zu ermöglichen. Es kann beispielsweise in den Erdboden eingesteckt, eingeschraubt oder eingehämmert sein. Im in den Erdboden eingebrachten Zustand kann sich ein 15 bis 100 cm langer Abschnitt, insbesondere ein 30 bis 50 cm langer Abschnitt des Messmoduls innerhalb des Erdbodens befinden. Zumindest Teile der Sensoren können auf verschiedenen Höhen des Messmoduls vorhanden sein, um beispielsweise eine Ermittlung eines vertikalen Flüssigkeitsflusses durch vertikal beabstandete Feuchtigkeitssensoren zu ermöglichen.

Das Messmodul kann eine Datenverarbeitungseinrichtung umfassen, die beispielsweise durch eine CPU bzw. einen Mikrokontroller mit zugeordnetem Speicher gebildet ist. Diese kann die Kommunikationseinrichtung steuern, den Sensor oder die Sensoren auslesen, optional das obig erläuterte Zwischenergebnis ermittelt oder Ähnliches. Das Gehäuse kann wasser- und/oder schmutzabweisend sein bzw. einen Aufnahmeraum für zumindest Teile der Elektronik, z. B. die Kommunikationseinrichtung, die Verarbeitungseinrichtung und/oder die Energieversorgungseinrichtung, umfassen, die wasser- bzw. schmutzabweisend sind. Hierbei soll insbesondere auch Einsickern von Wasser von der Bodenseite her durch eine entsprechende Gehäuseabdichtung verhindert werden. Das Gehäuse sollte die elektronischen Komponenten vor allen zu erwartenden Wetter- bzw. Jahreszeiteinflüssen schützen. Vorzugsweise ist das Gehäuse hinreichend stabil ausgebildet, dass es beispielsweise in den Boden eingehämmert werden kann bzw. dass eine Beschädigung von Komponenten, z.B. bei einem Überfahren des Messmoduls durch einen Traktor, verhindert wird.

Als Energieversorgungseinrichtung können eine Batterie und/oder ein Solarpanel genutzt werden. Die Kommunikationseinrichtung kann insbesondere zur Kommunikation über ein Mobilfunknetz oder ein Wide Area Network dienen. Verschiedene mögliche Protokolle hierfür wurden bereits obig diskutiert. An bzw. in dem Gehäuse kann auch eine Antenne für die Kommunikationseinrichtung vorgesehen sein.

Das Messmodul kann insbesondere eine Positionsbestimmungseinrichtung, z. B. ein GPS-Modul, umfassen. Um eine Konfiguration und/oder eine Statusprüfung am Messmodul selbst ohne zusätzliche Komponenten zu ermöglichen, können Statusleuchten, beispielsweise LEDs, Bedienelemente, beispielsweise Knöpfe, und/oder ein Display am Messmodul vorgesehen sein. Zudem kann das Messmodul eine Signaleinrichtung, beispielsweise ein helles Blinklicht oder eine akustische Hinweiseinrichtung, beispielsweise einen lauten Pieper, umfassen, die beispielsweise durch eine drahtlose Kommunikation aktiviert werden können, um ein Auffinden des Messmoduls zu erleichtern.

Weitere Vorteile und Einzelheiten der Erfindung ergeben sich aus den folgenden Ausführungsbeispielen sowie den zugehörigen Zeichnungen. Hierbei zeigen schematisch:

Fig. 1   ein Ausführungsbeispiel einer erfindungsgemäßen Vorrichtung zur Ermittlung einer Menge von gebundenem Kohlenstoffdioxid,

Fig. 2   das Zusammenwirken relevanter Algorithmen und Datenstrukturen im Rahmen eines Ausführungsbeispiels des erfindungsgemäßen Verfahrens zur Ermittlung der Menge des gebundenen Kohlenstoffdioxids, und

Fig. 3    eine beispielhafte Ausgestaltung des in Fig. 1 gezeigten Messmoduls.

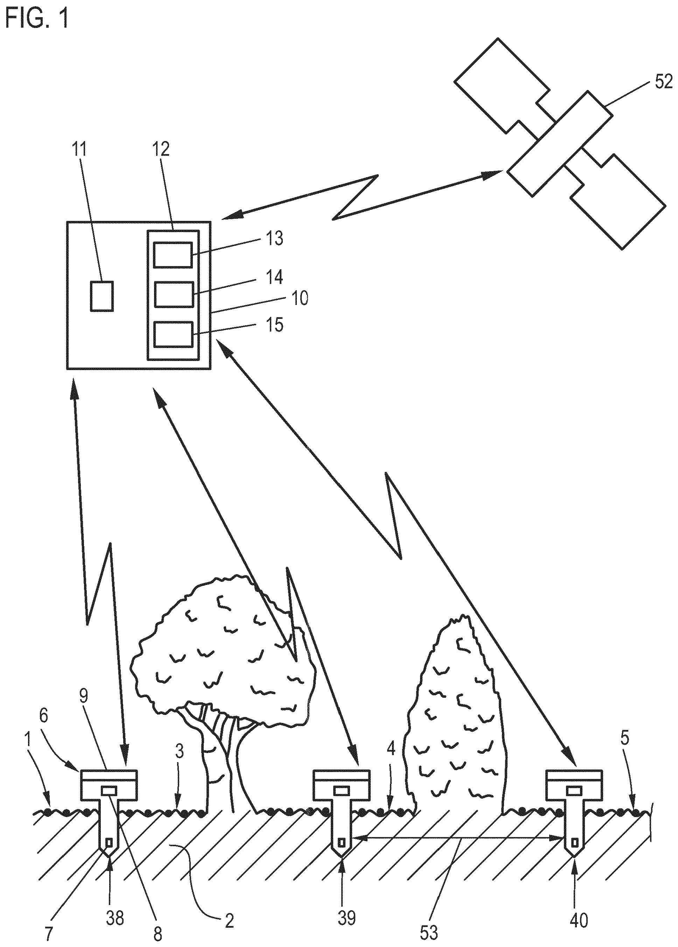

Fig. 1 zeigt schematisch eine Vorrichtung zur Ermittlung einer Menge von innerhalb eines Zeitintervalls mithilfe eines erdbodenseitigen Reaktionsmittels 1 chemisch gebundenem Kohlenstoffdioxid. Um eine übersichtliche Darstellung der relevanten Komponenten zu ermöglichen, weichen die relativen Größen bzw. Abstände zwischen den gezeigten Gegenständen teils deutlich von realen Größenverhältnissen bzw. Entfernungen ab.

Als Reaktionsmittel 1 wird fein gemahlenes Gestein genutzt, durch dessen Verwitterung, wie bereits im allgemeinen Teil erläutert, Kohlenstoffdioxid aus der Luft aufgenommen wird. Um eine möglichst große Reaktionsfläche bereitzustellen, ist das Reaktionsmittel 1 erdbodenseitig in mehreren Messgebieten 3, 4, 5, die im Beispiel separate, landwirtschaftlich genutzte Felder sind, großflächig verteilt. Hierbei hängt die Menge des im jeweiligen Messgebiet 3, 4, 5 gebundenen Kohlenstoffdioxids einerseits von Eigenschaften des Reaktionsmittels 1 selbst und andererseits von einer Vielzahl von weiteren Parametern, beispielsweise der Bodenbeschaffenheit, dem Wetter und Anderem, ab.

Um einen Anreiz für eine solche Kohlenstoffdioxidbindung zu schaffen, ist es hochrelevant, die gebundene Menge von Kohlenstoffdioxid zu quantifizieren, beispielsweise um die Bindung von Kohlenstoffdioxid im Rahmen eines Emissionszertifikathandels berücksichtigen zu können. Zwar könnte versucht werden, relevante Einflüsse alleine durch Entnahme von Bodenproben, die anschließend in einem Labor untersucht werden, zu berücksichtigen. Da für die Menge des gebundenen Kohlenstoffdioxids jedoch auch kurzzeitige Einflüsse, beispielsweise Regenschauer oder Wachstumsprozesse von Pflanzen, hochrelevant sind, wäre eine sehr häufige Untersuchung von Bodenproben erforderlich, was zu hohen Kosten führen würde und somit wirtschaftlich nicht zweckmäßig wäre.

Im gezeigten Beispiel wird daher ein anderes Verfahren genutzt, um die Menge des gebundenen Kohlenstoffdioxids zu ermitteln. Hierbei werden erdbodenseitige Sensoren 7 genutzt, um z.B. Messwerte für die Leitfähigkeit des Erdbodens zu erfassen, in Abhängigkeit derer die Menge des gebundenen Kohlenstoffdioxids ermittelt wird. Die Nutzung von lokalen Sensoren ermöglicht es, relevante Messwerte mit hoher zeitlicher und räumlicher Auflösung zu erfassen, so dass sowohl räumlich als auch zeitlich eine höhere Auflösung bereitgestellt werden kann, als dies mit vertretbarem Aufwand durch eine Entnahme von Bodenproben möglich wäre.

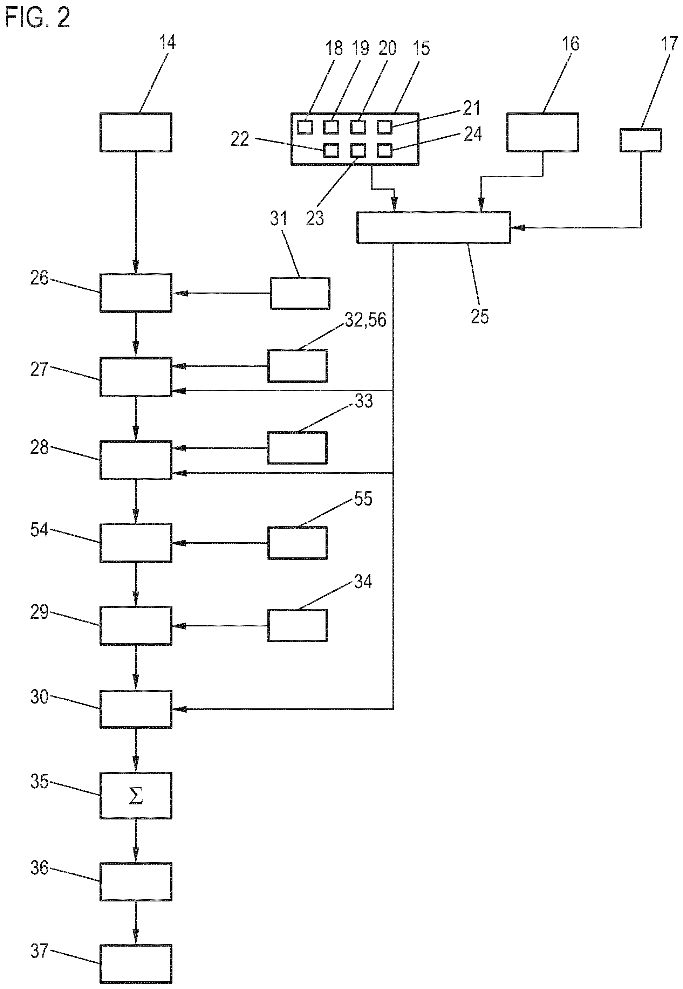

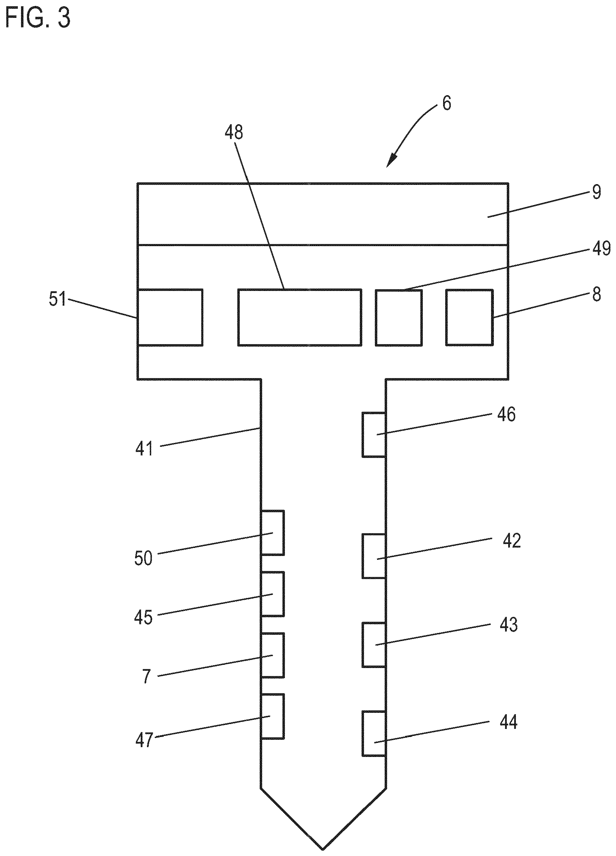

Ergänzend oder alternativ zur Erfassung der Leitfähigkeit des Erdbodens 2 kann auch eine Kationenkonzentration wenigstens eines Kations im Erdboden 2 als Messwert ermittelt werden bzw. es können ergänzend auch andere Sensordaten, die im Folgenden noch mit Bezug auf die Fig. 2 erläutert werden, berücksichtigt werden. Aus Übersichtlichkeitsgründen ist in Fig. 1 pro Messgebiet 3, 4, 5 bzw. pro dort jeweils angeordnetem Messmodul 6 jedoch nur genau sein Sensor 7 dargestellt. Ein Messmodul 6 mit größerer Sensorenzahl wird später noch mit Bezug auf Fig. 3 erläutert werden.

Ein mögliches Vorgehen zur Ermittlung der Menge des gebundenen Kohlenstoffdioxids wird im Folgenden mit zusätzlich in Bezug auf Fig. 2 erläutert, die das Zusammenwirken hierfür relevanten Algorithmen und Datenstrukturen zeigt. Hierbei erfolgt die Durchführung der Berechnungen durch eine Verarbeitungseinrichtung 10, bei der es sich beispielsweise um einen Server handeln kann. Ein in einem Speicher 12 gespeichertes Computerprogramm 13 wird durch einen Prozessor 11 durchgeführt, um die Schritte des Verfahrens zu implementieren.

Hierbei wird zunächst ein das Reaktionsmittel 1 betreffender Reaktionsmittelparameter 14 eingelesen. Dieser kann bereits im Speicher 12 der Verarbeitungseinrichtung 10 vorliegen oder, beispielsweise über das Internet, von einem Server abgerufen werden oder Ähnliches. Im Beispiel wird davon ausgegangen, dass der Reaktionsmittelparameter eine Zeitableitung der Menge des durch das Reaktionsmittel chemisch gebundenen Kohlenstoffdioxids unter Laborbedingungen beschreibt. Diese kann beispielsweise in Vorversuchen für eine bestimmte Charge von Reaktionsmitteln oder für einen bestimmten Reaktionsmitteltyp ermittelt werden.

Der Reaktionsmittelparameter bzw. der Grundwert für die Zeitableitung der Menge des gebundenen Kohlenstoffdioxids wird anschießend mit mehreren Korrekturfaktoren 26 bis 30 multipliziert, wie bereits im allgemeinen Teil der Beschreibung erläutert wurde.

Vereinfachend soll zunächst davon ausgegangen werden, dass ausschließlich der Korrekturfaktor 26 verwendet wird, der dem im allgemeinen Teil diskutierten Korrekturfaktor $f_{Leitfähigkeit}$ entspricht. Hierzu wird ein Messwert 31 für die Leitfähigkeit des Erdbodens 2 durch den jeweiligen Sensor 7 des jeweiligen Messmoduls 6 erfasst und durch eine Kommunikationseinrichtung 8 des jeweiligen Messmoduls 6 drahtlos an die Verarbeitungseinrichtung 10 übertragen, wonach mithilfe einer durch das Programm 13 bereitgestellten Berechnungsvorschrift der Korrekturfaktor 26 berechnet und der Grundwert mit diesem multipliziert wird. Die Energieversorgung des Messmoduls 6 bzw. insbesondere des Sensors 7 und der Kommunikationseinrichtung 8 erfolgt hierbei durch eine Energieversorgungseinrichtung 9, im Beispiel durch eine Solarzelle.

Nach der Multiplikation des Grundwertes mit dem Korrekturfaktor 26, bzw. in der in Fig. 2 gezeigten komplexeren Ausgestaltung mit allen Korrekturfaktoren 26 bis 30, werden die realen Verwitterungsraten bzw. die Zeitableitungen der Menge des gebundenen Kohlenstoffdioxids für die verschiedenen Messgebiete 3, 4, 5 und für die aufeinanderfolgenden Zeitintervalle, für die sich der Korrekturfaktor 26 potentiell jeweils unterscheidet, zueinander addiert, um die Menge 36 des insgesamt im Zeitintervall gebundenen Kohlenstoffdioxids zu ermitteln. Diese kann über eine Ausgangsschnittstelle 37, beispielsweise über ein Webinterface, an Nutzer bereitgestellt werden bzw. an andere Verarbeitungseinrichtungen bereitgestellt werden, beispielsweise um automatisch Emissionszertifikate auf Basis des gebundenen Kohlenstoffdioxids bereitstellen zu können.

Um die Genauigkeit der Ermittlung der Menge 36 zu verbessern, ist es vorteilhaft, zusätzlich die weiteren Korrekturfaktoren 27 bis 30 oder zumindest Teile hiervon zu nutzen. Der Korrekturfaktor 27 entspricht dem im allgemeinen Teil diskutierten Korrekturfaktor $f_{Boden}$. Dieser hängt, wie dort bereits erläutert wurde, insbesondere von einem Messwert 32 für die Kationenkonzentration wenigstens eines Kations im Erdboden 2 ab. Insbesondere können Konzentrationen für jene Kationen gemessen werden, die zunächst Teil des Reaktionsmittels sind und durch Bindung des Kohlenstoffdioxids als Karbonat ausfallen.

Es kann vorteilhaft sein, zur Ermittlung der verschiedenen Messwerte verschiedene Sensoren zu nutzen. Ein Messmodul 6, das verschiedene Sensoren 7, 42 bis 47, 50, 51 umfasst, ist in Fig. 3 schematisch dargestellt und wird später noch genauer erläutert werden. Der Messwert 32 kann beispielsweise durch den Sensor 45 bereitgestellt werden, der beispielsweise mithilfe einer ionenselektiven Elektrode die Kationenkonzentration misst.

Der Korrekturfaktor 27 kann zusätzlich von Zusatzinformationen 15 abhängig sein, die beispielsweise vorab im Speicher 12 der Verarbeitungseinrichtung 10 hinterlegt sein können oder über eine nicht gezeigte dritte Einrichtung bereitgestellt werden können.

Die Vorabinformationen 15 können mehrfach vorhanden sein, beispielsweise einmal für jedes der Messgebiete 3, 4, 5. Um die Vorabinformation jeweils gemeinsam mit den Messwerten 31, 32 bzw. Sensordaten 33, 34, 55 für das jeweilige Messgebiet 3, 4, 5 verarbeiten zu können bzw. separate Mengen von gebundenem Kohlenstoffdioxid für die einzelnen Messgebiete 3, 4, 5 bestimmen zu können, sind die Vorabinformationen 15 jeweils einem Gebiet 18 zugeordnet bzw. für jedes Gebiet 18 separat bereitgestellt. In jedem Messgebiet 3, 4, 5 ist zudem eine Positionsbestimmungseinrichtung 49, beispielsweise ein GPS-Sensor, vorhanden, die insbesondere in das Messmodul 6 integriert sein kann, wie in Fig. 3 dargestellt ist. Die Positionsbestimmungseinrichtung 49 stellt über die Kommunikationseinrichtung 8 gemeinsam mit den Messwerten 31, 32 bzw. den Sensordaten 33, 34 eine Positionsinformation bereit, so dass durch ein Wahlmodul 25 jeweils jene Vorabinformationen 15 zur gemeinsamen Verarbeitung mit den Messwerten 31, 32 bzw. Sensordaten 33, 34 eines Messgebiets 3, 4, 5 ausgewählt werden können, deren Gebiet 18 dem jeweiligen Messgebiet 3, 4, 5 entsprich.

Der Korrekturfaktor 27 soll insbesondere Eigenschaften des Erdbodens 2 abbilden, weshalb zur Ermittlung des Korrekturfaktors 27 als Vorabinformation 15 insbesondere eine Porosität 20 und/oder ein Bodentyp 21 des Erdbodens 2 und/oder im Erdboden 2 vorhandene Pilze 22 und/oder Bakterien 23 und/oder eine Bepflanzung 24 des Erdbodens 2 bereitgestellt werden können. Diese Vorabinformationen 15 können beispielsweise dadurch ermittelt werden, dass Bodenproben und im Labor untersucht werden bzw. sie können manuell eingegeben werden, beispielsweise um eine ausgebrachte Saat als Bepflanzung 24 zu berücksichtigen.

Neben den gezeigten Abhängigkeiten kann der Korrekturfaktor 27 zusätzlich von einem Nährstoffgehalt des Erdbodens 2 abhängen, der z.B. über einen weiteren Sensor 50 ermittelt werden kann, der in Fig. 3 gezeigt ist. Dieser Sensor kann z.B. die Konzentration von Natrium-, Phosphor- und/oder Kaliumverbindungen bzw. - ionen messen, die durch erdbodenseitige Nährstoffe bereitgestellt werden.

Der Korrekturfaktor 28 entspricht dem im allgemeinen Teil diskutierten Korrekturfaktor $f_{Umwelt}$. Dieser soll allgemein das Wetter bzw. im Speziellen eine Temperatur und eine Niederschlagsmenge berücksichtigen. Im Beispiel kann die Temperatur durch den in Fig. 3 gezeigten Temperatursensor 46 erfasst werden. Die Niederschlagsmenge könnte prinzipiell auch lokal gemessen werden, im Beispiel wird hierfür jedoch ein Sensor 52 genutzt, der satellitenbasiert ist.

Da über einen solchen satellitenbasierten Sensor 52 Niederschlagsmengen für eine Vielzahl von Gebieten erfasst werden, können die durch den Sensor 52 bereitgestellten Sensordaten 16 ebenfalls durch das Auswahlmodul 25 verarbeitete werden, um mithilfe der Positionsinformation 17 geeignete Teildaten auszuwählen, die bei der Ermittlung des Korrekturfaktors 28 berücksichtigt werden.

Optional können bei der Ermittlung des Korrekturfaktors 28 oder zur Ermittlung eines weiteren Korrekturfaktors auch Sensordaten berücksichtigt werden, die einen Partialdruck von Kohlenstoffdioxid in der Luft oberhalb des Erdbodens 2 betreffen. Diese können beispielsweise über den in Fig. 3 gezeigten Sensor 51 erfasst werden.

Der Korrekturfaktor 54 entspricht dem im allgemeinen Teil erläuterten Korrekturfaktor $f_{Flüssigkeit}$. Dieser hängt von Sensordaten 55 ab, die durch die in Fig. 3 gezeigten Sensoren 42 bis 44 erfasst werden können. Die Sensoren 42 bis 44 sind Feuchtigkeitssensoren, womit die Sensordaten die Feuchtigkeit im Erdboden 2 beschreiben. Aufgrund der vertikalen Beabstandung der Sensoren 42 bis 44 kann durch diese auch ein Lysimeter implementiert werden und der vertikale Wasserfluss im Erdboden 2 als Teil der Sensordaten 55 ermittelt und berücksichtigt werden.

Der Korrekturfaktor 29 entspricht dem im allgemeinen Teil erläuterten Korrekturfaktor $f_{pH}$ und hängt von Sensordaten 34 ab, die den pH-Wert im Erdboden 2 betreffen und die z.B. über den in Fig. 3 gezeigten Sensor 47 erfasst werden können.

Der Korrekturfaktor 30 hängt von der Alkalinität 19 des Erdbodens 2 ab, die im gezeigten Beispiel als Vorabinformation 15 bereitgestellt wird. Alternativ könnte die Alkalität auch sensorisch erfasst werden. Der Korrekturfaktor 30 entspricht somit dem im allgemeinen Teil erläuterten Korrekturfaktor $f_{Alk}$.

Berechnungsvorschriften zur Berechnung der einzelnen Korrekturfaktoren 26 bis 30 aus den genannten Größen werden durch das Programm 13 vorgegeben. Sie können beispielsweise durch Testreihen im Labor ermittelt werden, wobei beispielsweise Look-up-Tabellen, eine Regressionsanalyse, statistische Analysen ein Maschinenlernen und Ähnliches genutzt werden können, um aus diesen Versuchsreihen Berechnungsvorschriften zu generieren.

Die Messwerte bzw. Sensordaten werden durch das jeweilige Messmodul 6 vorzugsweise mehrmals täglich bzw. mehrmals pro Stunde erfasst, um eine Ermittlung der Menge des gebundenen Kohlenstoffdioxids mit hoher Zeitauflösung zu ermöglichen. Da sich die relevanten Parameter lokal, beispielsweise zwischen unterschiedlichen Feldern, unterscheiden können, kann, wie in Fig. 1 schematisch dargestellt ist, pro Feld ein separates Messmodul 6 genutzt werden. Allgemein kann der Abstand 53 zwischen Messpositionen 38 bis 40 bzw. Messmodulen 6 vorzugsweise kleiner als 500 m sein.

Bezüglich Fig. 1 wurde davon ausgegangen, dass die Energieversorgungseinrichtung 1 eine Solarzelle ist. Um beispielsweise auch Messungen nachts oder bei schlechtem Wetter zu ermöglichen, ist es vorteilhaft, wenn ergänzend oder alternativ als Energieversorgungseinrichtung 48 eine Batterie genutzt wird, wie in Fig. 3 schematisch dargestellt ist.

Sowohl in dem in Fig. 1 als auch in dem in Fig. 3 dargestellten Messmodul 6 wird jeweils davon ausgegangen, dass die Kommunikationseinrichtung 8 auch das Auslesen der verschiedenen Sensoren 7, 42-47, 50, 51, die Steuerung der Stromversorgung und unter Umständen eine Vorverarbeitung der Sensordaten zur Ermittlung von Zwischenergebnissen implementiert. Prinzipiell ist es auch möglich, entsprechende Funktionen durch eine separate, in das Messmodul 6 integrierte Verarbeitungseinrichtung, beispielsweise eine CPU mit zugeordnetem Speicher, zu implementieren.

Die elektronischen Komponenten des Messmoduls können zumindest Teilweise in ein Gehäuse 41 integriert sein, dass die Komponenten einerseits vor Feuchtigkeit schützen kann und/oder andererseits eine Beschädigung der Komponenten, z.B. bei einem Einhämmern in den Erdboden oder auch bei einem überfahren, z.B. durch einen Traktor, verhindert.

**Patentansprüche**

1. Verfahren zur Ermittlung einer Menge (36) von innerhalb eines Zeitintervalls mithilfe eines erdbodenseitigen Reaktionsmittels (1) chemisch gebundenem Kohlenstoffdioxid, umfassend die Schritte:

   - Einlesen wenigstens eines das Reaktionsmittel (1) betreffenden Reaktionsmittelparameters (14),
   - Erfassen eines jeweiligen Messwertes (31, 32) für die Leitfähigkeit des Erdbodens (2) und/oder für eine jeweilige Kationenkonzentration wenigstens eines Kations im Erdboden (2) durch wenigstens einen in oder an dem Erdboden (2) angebrachten Sensor (7, 45), und
   - Ermittlung der Menge des gebundenen Kohlenstoffdioxids in Abhängigkeit sowohl des Reaktionsmittelparameters (14) als auch des Messwertes (31, 32) oder der Messwerte (31, 32).

2. Verfahren nach Anspruch 1, **dadurch gekennzeichnet, dass** die Menge (36) des gebundenen Kohlenstoffdioxids in Abhängigkeit von Sensordaten (33, 55, 56) des Sensors (7, 45) und/oder wenigstens eines weiteren Sensors (42-44, 50, 52, 57) ermittelt wird, wobei die Sensordaten (33, 55, 56) einen vertikalen Wasserfluss im Erdboden (2) und/oder eine Niederschlagsmenge und/oder eine Feuchtigkeit im Erdboden (2) und/oder eine Alkalinität und/oder einen Nährstoffgehalt des Erdbodens (2) und/oder einen Partialdruck von Kohlenstoffdioxid in der Luft oberhalb des Erdbodens (2) betreffen.

3. Verfahren nach Anspruch 1 oder 2, **dadurch gekennzeichnet, dass** die Menge (36) des gebundenen Kohlenstoffdioxids in Abhängigkeit von Sensordaten (33, 34) des Sensors (7, 45) und/oder des weiteren Sensors (42-44, 50, 52, 57) und/oder wenigstens eines weiteren Sensors (46, 51) ermittelt wird, wobei die Sensordaten (33, 34) einen pH-Wert und/oder eine Temperatur des Erdbodens (2) betreffen.

4. Verfahren nach einem der vorangehenden Ansprüche, **dadurch gekennzeichnet, dass** der Reaktionsmittelparameter (14) einen Grundwert für die Menge (36) des gebundenen Kohlenstoffdioxids oder für die Zeitableitung dieser Menge vorgibt, der im Rahmen der Berechnung mit wenigstens einem Korrekturfaktor (26-30, 54) multipliziert wird, wobei wenigstens einer der Korrekturfaktoren (26-30, 54) jeweils von dem Messwert (31, 32) oder wenigstens einem der Messwerte (31, 32) und/oder von zumindest Teilen der Sensordaten (33, 34, 55, 56) abhängt.

5. Verfahren nach einem der vorangehenden Ansprüche, **dadurch gekennzeichnet, dass** als Kationenkonzentration die Konzentration von Natriumionen und/oder von Kalziumionen und/oder von Magnesiumionen erfasst wird.

6. Verfahren nach einem der vorangehenden Ansprüche, **dadurch gekennzeichnet, dass** der jeweilige Messwert (31, 32) und/oder die Sensordaten (33, 34, 55, 56) und/oder wenigstens ein aus dem Messwert (31, 32) und/oder den Sensordaten (33, 34, 55, 56) ermitteltes Zwischenergebnis von einer Messposition (38, 39, 40), an der der jeweilige Sensor (7, 42-47, 50, 51) oder die jeweiligen Sensoren (7, 42-47, 50, 51) angeordnet ist oder sind, drahtlos an eine Verarbeitungseinrichtung (10) übertragen wird, die die Ermittlung der Menge (36) des gebundenen Kohlenstoffdioxids durchführt.

7. Verfahren nach einem der vorangehenden Ansprüche, **dadurch gekennzeichnet, dass** mehrmals täglich oder mehrmals pro Stunde der Messwert (31, 32) oder die Messwerte (31, 32) und/oder zumindest Teile der Sensordaten (33, 34, 55, 56) erfasst werden und in Abhängigkeit hiervon die Menge (36) des gebundenen Kohlenstoffdioxids ermittelt wird.

8. Verfahren nach einem der vorangehenden Ansprüche, **dadurch gekennzeichnet, dass** der Messwert (31, 32) oder wenigstens einer der Messwerte (31, 32) und/oder die Sensordaten (33, 34, 55, 56) an mehreren Messpositionen (38, 39, 40) durch einen jeweiligen Sensor (7, 42-47, 50, 51) erfasst werden, wobei die Messpositionen (38, 39, 40) insbesondere weniger als 500 m oder weniger als 200 m voneinander beabstandet sind.

9. Verfahren nach einem der vorangehenden Ansprüche, **dadurch gekennzeichnet, dass** dem Erdboden (2), insbesondere im Bereich einer jeweiligen Messposition (38, 39, 40), an der der jeweilige Sensor (7, 42-47, 50, 51) oder die jeweiligen Sensoren (7, 42-47, 50, 51) angeordnet ist oder sind, vor Erfassung des jeweiligen Messwertes (31, 32) und/oder der Sensordaten (33, 34, 55, 56) eine Bodenprobe entnommen wird, die, insbesondere beabstandet von dem Entnahmeort in einem Labor, untersucht wird, um eine Alkalinität (19) und/oder eine Porosität (20) und/oder einen Bodentyp (21) des Erdbodens (2) und/oder im Erdboden (2) vorhandene Pilze (22) und/oder Bakterien (23) als Vorabinformation (15) zu ermitteln, wobei die Menge (36) des gebundenen Kohlenstoffdioxids in Abhängigkeit der Vorabinformation (15) ermittelt wird.

10. Verfahren nach einem der vorangehenden Ansprüche, **dadurch gekennzeichnet, dass** eine jeweilige Vorabinformation (15), die insbesondere eine Alkalinität (19) und/oder eine Porosität (20) und/oder einen Bodentyp (21) des Erdbodens (2) und/oder im Erdboden (2) vorhandene Pilze (22) und/oder Bakterien (23) und/oder eine Bepflanzung (24) des Erdbodens (2) und/oder das Wetter betrifft, und/oder die jeweilige auf Basis der Bodenprobe bestimmte Vorabinformation (15) für mehrere Gebiete (18) separat bereitgestellt wird, wobei in einem Messgebiet (3, 4, 5), das den und/oder den weiteren Sensor (7, 42-47, 50, 51) umfasst, zusätzlich eine Positionsbestimmungseinrichtung (49) verwendet wird, die eine das jeweilige Messgebiet (3, 4, 5) betreffende Positionsinformation (17) bereitstellt, wobei auf Basis der Positionsinformation (17) die dem Messgebiet (3, 4, 5) zugeordnete Vorabinformation (15) ausgewählt wird, wonach in Abhängigkeit von dieser und dem wenigstens einen in dem Messgebiet (3, 4, 5) erfassten Messwert (32, 33) und/oder von zumindest Teilen der in dem Messgebiet (3, 4, 5) erfassten Sensordaten (33, 34, 55, 56) die Menge (36) des gebundenen Kohlenstoffdioxids oder ein Zwischenergebnis, von dem die ermittelte Menge (36) des gebundenen Kohlenstoffdioxids abhängt, bestimmt wird.

11. Vorrichtung zur Ermittlung einer Menge (36) von innerhalb eines Zeitintervalls mithilfe eines erdbodenseitigen Reaktionsmittels (1) chemisch gebundenem Kohlenstoffdioxid, umfassend wenigstens einen Sensor (7, 45) zur Erfassung von Messwerten (31, 32) für die Leitfähigkeit des Erdbodens (2) und/oder für eine jeweilige Kationenkonzentration wenigstens eines Kations im Erdboden (2) und eine Verarbeitungseinrichtung (10), **dadurch gekennzeichnet, dass** die Vorrichtung zur Durchführung des Verfahrens nach einem der vorangehenden Ansprüche eingerichtet ist.

12. Vorrichtung nach Anspruch 11, **dadurch gekennzeichnet, dass** sie wenigstens ein Messmodul (6) umfasst, das ein Gehäuse (41), das zur Einführung in den Erdboden (2) ausgebildet ist, wenigstens einen Sensor (7, 42-47, 50, 51), eine Kommunikationseirichtung (8) zur drahtlosen Übertragung von mittels des Sensors (7, 42-47, 50, 51) erfassten Messwerten (31, 32) und/oder Sensordaten (33, 34, 55, 56) und/oder eines aus dem Messwert (31, 32) und/oder den Sensordaten (33, 34, 55, 56) ermittelten Zwischenergebnisses an die Verarbeitungseinrichtung (10) und wenigstes eine Energieversorgungseinrichtung (9, 48) zur Versorgung der Kommunikationseinrichtung (8) und des Sensors (7, 42-47, 50, 51) umfasst.

## Claims

1. Method for determining an amount (36) of carbon dioxide chemically bound within a time interval using a reactant (1) on the ground, comprising the steps of:

- reading in at least one reactant parameter (14) concerning the reactant (1),
- capturing a respective measured value (31, 32) for the conductivity of the ground (2) and/or for a respective cation concentration of at least one cation in the ground (2) by means of at least one sensor (7, 45) mounted in or on the ground (2), and
- determining the amount of bound carbon dioxide on the basis of both the reactant parameter (14) and the measured value (31, 32) or the measured values (31, 32).

2. Method according to Claim 1, **characterized in that** the amount (36) of bound carbon dioxide is determined on the basis of sensor data (33, 55, 56) from the sensor (7, 45) and/or at least one further sensor (42-44, 50, 52, 57), wherein the sensor data (33, 55, 56) relate to a vertical flow of water in the ground (2) and/or an amount of precipitation and/or moisture in the ground (2) and/or an alkalinity and/or a nutrient content of the ground (2) and/or a partial pressure of carbon dioxide in the air above the ground (2).

3. Method according to Claim 1 or 2, **characterized in that** the amount (36) of bound carbon dioxide is determined on the basis of sensor data (33, 34) from the sensor (7, 45) and/or the further sensor (42-44, 50, 52, 57) and/or at least one further sensor (46, 51), wherein the sensor data (33, 34) relate to a pH value and/or a temperature of the ground (2).

4. Method according to one of the preceding claims, **characterized in that** the reactant parameter (14) specifies a basic value for the amount (36) of bound carbon dioxide or for the time derivative of this amount, which is multiplied in the course of the calculation by at least one correction factor (26-30, 54), wherein at least one of the correction factors (26-30, 54) depends in each case on the measured value (31, 32) or at least one of the measured values (31, 32) and/or at least parts of the sensor data (33, 34, 55, 56).

5. Method according to one of the preceding claims, **characterized in that** the concentration of sodium ions and/or calcium ions and/or magnesium ions is captured as the cation concentration.

6. Method according to one of the preceding claims, **characterized in that** the respective measured value (31, 32) and/or the sensor data (33, 34, 55, 56) and/or at least one intermediate result determined from the measured value (31, 32) and/or the sensor data (33, 34, 55, 56) from a measuring position (38, 39, 40), at which the respective sensor (7, 42-47, 50, 51) or the respective sensors (7, 42-47, 50, 51) is or are arranged, is/are transmitted wirelessly to a processing device (10) which determines the amount (36) of bound carbon dioxide.

7. Method according to one of the preceding claims, **characterized in that** the measured value (31, 32) or the measured values (31, 32) and/or at least parts of the sensor data (33, 34, 55, 56) is/are captured several times a day or several times per hour and the amount (36) of bound carbon dioxide is determined on the basis of this.

8. Method according to one of the preceding claims, **characterized in that** the measured value (31, 32) or at least one of the measured values (31, 32) and/or the sensor data (33, 34, 55, 56) at a plurality of measuring positions (38, 39, 40) is/are captured by a respective sensor (7, 42-47, 50, 51), wherein the measuring positions (38, 39, 40) are in particular less than 500 m or less than 200 m apart.

9. Method according to one of the preceding claims, **characterized in that** a soil sample is taken from the ground (2), in particular in the region of a respective measuring position (38, 39, 40), at which the respective sensor (7, 42-47, 50, 51) or the respective sensors (7, 42-47, 50, 51) is or are arranged, prior to capturing the respective measured value (31, 32) and/or the sensor data (33, 34, 55, 56), and is examined, in particular at a distance from the sampling location in a laboratory, in order to determine an alkalinity (19) and/or a porosity (20) and/or a soil type (21) of the ground (2) and/or fungi (22) and/or bacteria (23) present in the ground (15) as preliminary information (2), wherein the amount (36) of bound carbon dioxide is determined on the basis of the preliminary information (15).

10. Method according to one of the preceding claims, **characterized in that** respective preliminary information (15), in particular relating to an alkalinity (19) and/or a porosity (20) and/or a soil type (21) of the ground (2) and/or fungi (22) and/or bacteria (23) present in the ground (2) and/or planting (24) of the ground (2) and/or the weather, and/or the respective preliminary information (15) determined on the basis of the soil sample is provided separately for a plurality of areas (18), wherein in a measuring area (3, 4, 5), which comprises the sensor and/or the further sensor (7, 42-47, 50, 51), use is additionally made of a position determining device (49) which provides position information (17) relating to the respective measuring area (3, 4, 5), wherein the preliminary information (15) assigned to the measuring area (3, 4, 5) is selected on the basis of the position information (17), after which, on the basis of said preliminary information and the at least one measured value (32, 33) captured in the measuring area (3, 4, 5) and/or at least parts of the sensor data (33, 34, 55, 56) captured in the measuring area (3, 4, 5), the amount (36) of bound carbon dioxide or an intermediate result, on which the determined amount (36) of bound carbon dioxide depends, is determined.

11. Apparatus for determining an amount (36) of carbon dioxide chemically bound within a time interval using a reactant (1) on the ground, comprising at least one sensor (7, 45) for capturing measured values (31, 32) for the conductivity of the ground (2) and/or for a respective cation concentration of at least one cation in the ground (2) and a processing device (10), **characterized in that** the apparatus is configured to carry out the method according to one of the preceding claims.

12. Apparatus according to Claim 11, **characterized in that** it comprises at least one measuring module (6) comprising a housing (41), which is designed to be inserted into the ground (2), at least one sensor (7, 42-47, 50, 51), a communication device (8) for wirelessly transmitting measured values (31, 32) captured by means of the sensor (7, 42-47, 50, 51) and/or sensor data (33, 34, 55, 56) and/or an intermediate result determined from the measured value (31, 32) and/or the sensor data (33, 34, 55, 56) to the processing device (10), and at least one energy supply device (9, 48) for supplying the communication device (8) and the sensor (7, 42-47, 50, 51).

## Revendications

1. Procédé de détermination d'une quantité (36) de dioxyde de carbone lié chimiquement en un intervalle de temps à l'aide d'un réactif (1) dans le sol, comprenant les étapes de :

   - lecture d'au moins un paramètre (14) de réactif concernant le réactif (1),
   - détection d'une valeur de mesure (31, 32) respective pour la conductivité du sol (2) et/ou pour une concentration respective en cations d'au moins un cation dans le sol (2) par au moins un capteur (7, 45) posé dans ou au niveau du sol (2) et
   - détermination de la quantité de dioxyde de carbone lié en fonction aussi bien du paramètre (14) de réactif que de la valeur de mesure (31, 32) ou des valeurs de mesure (31, 32).

2. Procédé selon la revendication 1, **caractérisé en ce que** la quantité (36) du dioxyde de carbone lié est déterminée en fonction de données (33, 55, 56) du capteur (7, 45) et/ou d'au moins un capteur supplémentaire (42-44, 50, 52, 57), les données (33, 55, 56) de capteur concernant un flux d'eau vertical dans le sol (2) et/ou une quantité de précipitations et/ou une humidité dans le sol (2) et/ou une alcalinité et/ou une teneur en éléments nutritifs du sol (2) et/ou une pression partielle de dioxyde de carbone dans l'air au-dessus du sol (2).

3. Procédé selon la revendication 1 ou 2, **caractérisé en ce que** la quantité (36) du dioxyde de carbone lié est déterminée en fonction de données (33, 34) du capteur (7, 45) et/ou du capteur supplémentaire (42-44, 50, 52, 57) et/ou d'au moins un capteur supplémentaire (46, 51), les données (33, 34) de capteur concernant une valeur de pH et/ou une température du sol (2).

4. Procédé selon l'une des revendications précédentes, **caractérisé en ce que** le paramètre (14) de réactif prédéfinit une valeur de base pour la quantité (36) du dioxyde de carbone lié ou pour la dérivée temporelle de cette quantité, qui est multipliée dans le cadre du calcul par au moins un facteur de correction (26-30, 54), au moins l'un des facteurs de correction (26-30, 54) dépendant respectivement de la valeur de mesure (31, 32) ou d'au moins l'une des valeurs de mesure (31, 32) et/ou au moins de parties des données (33, 34, 55, 56) de capteur.

5. Procédé selon l'une des revendications précédentes, **caractérisé en ce qu'**on détecte, comme concentration en cations, la concentration en ions de sodium et/ou en ions de calcium et/ou en ions de magnésium.

6. Procédé selon l'une des revendications précédentes, **caractérisé en ce que** la valeur de mesure (31, 32) respective et/ou les données (33, 34, 55, 56) de capteur et/ou au moins un résultat intermédiaire, déterminé à partir de la valeur de mesure (31, 32) et/ou des données (33, 34, 55, 56) de capteur, d'une position de mesure (38, 39, 40), dans laquelle le capteur (7, 42-47, 50, 51) respectif ou les capteurs (7, 42-47, 50, 51) respectifs est/sont agencés, est/sont transmis(es) sans fil un appareil de traitement (10) qui réalise la détermination de la quantité (36) du dioxyde de carbone lié.

7. Procédé selon l'une des revendications précédentes, **caractérisé en ce que** la valeur de mesure (31, 32) ou les valeurs de mesure (31, 32) et/ou au moins des parties des données (33, 34, 55, 56) de capteur sont détectées plusieurs fois par jour ou plusieurs fois par heure et la quantité (36) du dioxyde de carbone lié est déterminée en fonction de celles-ci.

8. Procédé selon l'une des revendications précédentes, **caractérisé en ce que** la valeur de mesure (31, 32) ou au moins l'une des valeurs de mesure (31, 32) et/ou les données (33, 34, 55, 56) de capteur est/sont détectée(s) en plusieurs positions de mesure (38, 39, 40) par un capteur (7, 42-47, 50, 51) respectif, les positions de mesure (38, 39, 40) étant éloignées les unes des autres en particulier de moins de 500 m ou de moins de 200 m.

9. Procédé selon l'une des revendications précédentes, **caractérisé en ce qu'**un échantillon de sol est prélevé du sol (2), en particulier dans la zone d'une position de mesure (38, 39, 40) respective, dans laquelle le capteur (7, 42-47, 50, 51) respectif ou les capteurs (7, 42-47, 50, 51) est/sont agencés, avant la détection de la valeur de mesure (31, 32) respective et/ou des données (33, 34, 55, 56) de capteur, lequel échantillon est analysé, en particulier à distance du site de prélèvement dans un laboratoire, afin de déterminer une alcalinité (19) et/ou une porosité (20) et/ou un type (21) du sol (2) et/ou des champignons (22) et/ou des bactéries (23) présent(e)s dans le sol (2) en tant qu'information préalable (15), la quantité (36) du dioxyde de carbone lié étant déterminée en fonction de l'information préalable (15).

10. Procédé selon l'une des revendications précédentes, **caractérisé en ce qu'**une information préalable (15) respective, qui concerne en particulier une alcalinité (19) et/ou une porosité (20) et/ou un type (21) du sol (2) et/ou des champignons (22) et/ou des bactéries (23) présent(e)s dans le sol (2) et/ou une plantation (24) du sol (2) et/ou les conditions météorologiques et/ou l'information préalable (15) respective déterminée sur la base de l'échantillon du sol est/sont mise(s) à disposition séparément pour différentes zones (18), un dispositif de détermination de position (49), qui met à disposition une information de position (17) concernant la zone de mesure (3, 4, 5) respective, étant utilisé en plus dans une zone de mesure (3, 4, 5) qui comprend le capteur et/ou le capteur supplémentaire (7, 42-47, 50, 51) l'information préalable (15) associée à la zone de mesure (3, 4, 5) étant choisie sur la base de l'information de position (17), après quoi, en fonction de celle-ci et de ladite au moins une valeur de mesure (32, 33) détectée dans la zone de mesure (3, 4, 5) et/ou au moins de parties des données (33, 34, 55, 56) de capteur détectées dans la zone de mesure (3, 4, 5), la quantité (36) du dioxyde de carbone lié ou un résultat intermédiaire, dont dépend la quantité déterminée (36) du dioxyde de carbone lié, est déterminé(e).

11. Dispositif de détermination d'une quantité (36) de dioxyde de carbone lié chimiquement en un intervalle de temps à l'aide d'un réactif (1) dans le sol, comprenant au moins un capteur (7, 45), destiné à détecter des valeurs de mesure (31, 32) pour la conductivité du sol (2) et/ou pour une concentration respective en cations d'au moins un cation dans le sol (2), et un appareil de traitement (10), **caractérisé en ce que** le dispositif est conçu pour la mise en œuvre du procédé selon l'une des revendications précédentes.

12. Dispositif selon la revendication 11, **caractérisé en ce qu'**il comprend au moins un module de mesure (6), qui comprend un boîtier (41), qui est conçu pour être introduit dans le sol (2), au moins un capteur (7, 42-47, 50, 51), un appareil de communication (8), destiné à transmettre sans fil des valeurs de mesure (31, 32) détectées au moyen du capteur (7, 42-47, 50, 51) et/ou des données (33, 34, 55, 56) de capteur et/ou un résultat intermédiaire déterminé à partir de la valeur de mesure (31, 32) et/ou des données (33, 34, 55, 56) de capteur à l'appareil de traitement (10), et au moins un appareil d'alimentation en énergie (9, 48) destiné à alimenter l'appareil de communication (8) et le capteur (7, 42-47, 50, 51).

FIG. 1

FIG. 2

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2020263910 A1

**In der Beschreibung aufgeführte Nicht-Patentliteratur**

- **BARGRIZAN SIMA et al.** Constraining the carbonate system in soils via testing the internal consistency of pH, pCO2 and alkalinity measurements. *GEOCHEMICAL TRANSACTIONS*, 22 April 2020, vol. 21